# EUROPEAN PATENT APPLICATION

(11) **EP 1 553 167 A1**
(43) Date of publication of application: **13.07.2005**
(21) Application number: 04078279.9
(22) Date of filing: 03.12.2004
(51) Int. Cl.: C12N 1/06, C12P 1/00

(54) **Method for the production of a fermentation product from an organism**

(30) Priority: 30.12.2003 NL 1025149
(71) Applicant: Technische Universiteit Delft, 2628 BL Delft (NL)
(72) Inventor: Van Hee, Pim, 2613 TV Delft (NL); Van der Wielen, Lucas A. M., 2665 AR Bleiswijk (NL); Van der Lans, Robert G. J. M., 2641 ZH Pijnacker (NL)
(74) Representative: Kupecz, Arpad

(57) **Abstract**

The invention relates to a method for the production of a fermentation product from an organism cultured in a culture medium, wherein the organism after the formation of the fermentation product is subjected to a lysis treatment in the presence of a lysis-promoting compound. In accordance with the invention, the lysis-promoting agent is a compound that can be metabolised by the organism, and the method comprises the steps of treating the organism at a first, relatively high concentration of the lysis-promoting compound, separating the lysis-promoting agent and the fermentation product, and culturing the organism at a second, relatively low concentration of the metabolisable lysis-promoting agent.

## Description

The present invention relates to a method for the production of a fermentation product from an organism, wherein the organism is first cultured in a culture medium for the formation of the fermentation product and subsequently subjected to a lysis treatment in the presence of a lysis-promoting compound.

Such a method is generally known. Although the aim in many cases is to excrete the fermentation product into the culture medium via the organism, this is not always feasible or desirable. In such cases the organism has to undergo lysis. This occurs mechanically, for example by means of a pressure drop, shearing, or grinding in the presence of hard particles. In order to promote lysis, a lysis-promoting compound may be present. Harrison, T.L. et al.(Bioseparation 2: pp. 95-105) describe the use of sodium hydroxide, SDS, lysozyme and EDTA.

The addition of such a compound is accompanied by costs and reuse is rarely or never possible.

It is the object of the present invention to provide an improvement, with which the production costs of the fermentation product are reduced.

To this end the method according to the invention is characterized in that the lysis-promoting agent is a compound that can be metabolised by the organism, which method comprises the steps of
a) lysis of the organism at a first, relatively high concentration of the lysis-promoting compound,
b) separation of the lysis-promoting agent and the fermentation product, and
c) culture of the organism at a second, relatively low concentration of the metabolisable lysis-promoting agent.

In thus way, after the lysis-promoting compound has served its purpose, it can be used for the formation of additional fermentation product. The invention is in particular suitable for organisms chosen from bacteria, especially Gram-negative bacteria, as well as yeasts and fungi.

The lysis treatment is preferably a mechanical lysis treatment.

Such a treatment, furthered by the measures according to the invention, avoids the introduction of additional chemicals, as would be the case with a chemical lysis treatment.

According to a first embodiment, the fermentation product is separated from a fraction, which in addition to the metabolisable lysis-promoting compound contains other cell components of the organism, and the fraction is used in step c for culturing the organism.

In this way other cell components, such as cell proteins and the like, can be used for forming additional fermentation product, at least for those organisms that are able to metabolise such other cell components. This is especially applicable for many types of bacteria.

According to an important embodiment, the metabolisable lysis-promoting compound is an ammonium compound.

When using an ammonium compound, ammonia can be separated from the fermentation product by means of evaporation, for example at reduced pressure. Many organisms are able to use the ammonia as nitrogen source.

According to a preferred embodiment, the metabolisable lysis-promoting compound is ammonium hydroxide.

When the ammonium hydroxide separates from the fermentation product, there is no residue.

It is preferred for the ammonia to be removed from the fermentation product subjected to lysis by injecting a gas.

The gas is preferably a gas that is used for the culture of the organism, advantageously air, or possibly gas comprising carbon dioxide derived from the fermentation.

The invention will now be elucidated by way of the exemplary embodiment given below, wherein
Fig. 1 shows the results of a first experiment using the method according to the invention, and
Fig. 2 shows the results from a second experiment using the method according to the invention.

### EXAMPLE

### Experimental procedure for the demonstration of cell weakening owing to additive

### Fermentation

In accordance with the method of Weusthuis et al. (Ref. 1) Pseudomonas putida KT2442 was cultured in 8 litres of medium in a 10-litre fed-batch fermentor at 30°C. During fermentation, the pH was maintained at 7 using 25 vol./vol.% of ammonium hydroxide solution. The carbon source in the culture consisted of free fatty acids obtained from coconut oil (Vereenigde Oliefrabrieken, Rotterdam, the Netherlands) in a concentration of 0.4 vol./vol.% per litre of medium, yielding 131 g dry bacteria mass per litre of medium. This carbon source causes the organism to accumulate polyhydroxy alkanoate inclusion bodies. After fermentation, the batter was stored for a maximum of 3 months at 4-8°C.

### Cell disruption

250 ml of fermentation batter of Pseudomonas putida KT2442 was stirred for 1 hour using a magnetic agitator. Subsequently the desired pH for the pre-treatment was raised to pH = 11 using 25 vol./vol.% ammonium hydroxide solution. Fermentation batter that was not treated with ammonium hydroxide was used also, as reference measurement. After adjusting the pH, the mixture was stirred for 30 minutes using a magnetic agitator, whereafter it was homogenised with the aid of a homogeniser (Constant Cell Disruption Systems, Low March, Leerdam, the Netherlands) having a cell volume of 10 ml. The homogeniser was operated at a temperature of 10°C and the mixture to be homogenised was cooled between homogenisation passages. The number of homogenisation passages was varied from 0 to 5 and the effect of the homogenisation pressure in 1 passage was assessed at 1.0, 1.3, 1.6 or 2.0 kbar.

### Test for effectiveness of the cell disruption

Homogenised samples were centrifuged at 30,000 x g for 3 hours in order to retain the inclusion bodies (density approximately 1,000 kg/m3) in the supernatant and to pelletise the remaining cell residue (density approximately 1,085 kg/m3). After centrifugation, pellet and supernatant were separated with the aid of a glass Pasteur pipette. Both phases were lyophilised for 48 hours, whereafter the dry matter was weighed and the content of inclusion bodies was determined by means of gas chromatography according to the technique of De Rijk et al. (Ref. 2). With the aid of these analyses, the total amount of inclusion bodies in the supernatant was determined (free inclusion bodies), as well as the total amount of inclusion bodies in the pellet (inclusion bodies that are not free). The ratio of these two values is a measure for the effectiveness of cell disruption.

### Results

### 1. Effect of pressure on cell disruption

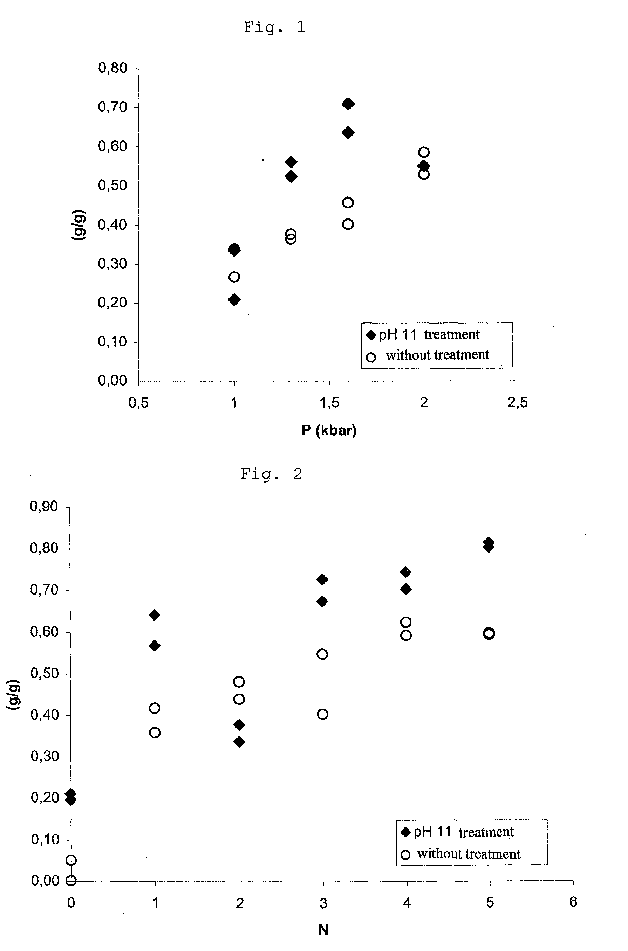

The effectiveness of cell disruption by means of homogenising at different pressures and homogenisation passages with and without ammonium hydroxide pre-treatment was examined by measuring the fraction of inclusion bodies released from the cells. In Fig. 1, the results from examining the relationship between the homogenisation pressure and the fraction of free inclusion bodies are represented for fermentation batter with and without ammonium hydroxide treatment. More specifically, the graph in Fig. 1 shows that in comparison with cells that were not subjected to treatment with ammonium hydroxide, the treatment with ammonium hydroxide at pH 11 ensures that under identical homogenisation conditions, more inclusion bodies are released from the cells.

### 2. Effect of the number of homogenisation steps (N) on cell disruption

The effectiveness of cell disruption by means of homogenisation at a constant pressure and different homogenisation steps with and without ammonium hydroxide pre-treatment was again examined by measuring the fraction of inclusion bodies released from the cells. The result is shown in the graph of Fig. 2.

The data again show that in comparison with homogenisation without pre-treatment, the release of inclusion bodies is facilitated by treatment with ammonium hydroxide at pH 11.

Treatment with ammonium hydroxide at pH 11 releases a fraction of the inclusion bodies into the supernatant even at zero passages (N). This phenomenon occurs only with cells that have been stored for some time at 4-8°C. In this case the cells had been stored for 9 weeks before the experiment was performed. This does not detract from the observable fact that an ammonium hydroxide treatment weakens the cells, seeing that the untreated cells had also been stored for 9 weeks.

### Conclusion

The homogenisation experiments show that a treatment of the cells with ammonium hydroxide facilitates the release of inclusion bodies with the aid of homogenisation, and thus makes cell disruption more effective.

### Literature

1. Weusthuis, R.A. et al.(2001) Biopolymers 3a: Polyesters I: Biological Systems and Biotechnological Production, Wiley-VCH, pp. 291-316.
2. De Rijk, T.C. et al.(2001) Biopolymers volume 3b: Polyesters II: Properties and Chemical Synthesis, Wiley-VCH, p. 1.

## Claims

1. A method for the production of a fermentation product from an organism, wherein the organism is first cultured in a culture medium for the formation of the fermentation product and is subsequently subjected to a lysis treatment in the presence of a lysis-promoting compound, **characterised in that** the lysis-promoting agent is a compound that can be metabolised by the organism, which method comprises the steps of
a) a)lysis of the organism at a first, relatively high concentration of the lysis-promoting compound,
b) separation of the lysis-promoting agent and the fermentation product, and
c) culture of the organism at a second, relatively low concentration of the metabolisable lysis-promoting agent.

2. A method according to claim 1, **characterised in that** the lysis treatment is a mechanical lysis treatment.

3. A method according to claim 1 or 2, **characterised in that** the fermentation product is separated from a fraction, which in addition to the metabolisable lysis-promoting compound contains other cell components of the organism, and the fraction is used in step c for culturing the organism.

4. A method according to one of the preceding claims, **characterised in that** the metabolisable lysis-promoting compound is an ammonium compound.

5. A method according to one of the preceding claims, **characterised in that** the metabolisable lysis-promoting compound is ammonium hydroxide.

6. A method according to one of the preceding claims, **characterised in that** the removal of the ammonia from the fermentation product subjected to lysis occurs by injecting a gas.
